# EUROPEAN PATENT SPECIFICATION

(11) **EP 3 731 836 B1**
(45) Date of publication and mention of the grant of the patent: **20.09.2023**
(21) Application number: 18830851.4
(22) Date of filing: 27.12.2018
(51) Int. Cl.: A61K 31/138, A61K 31/00, A61K 31/166, A61K 31/18, A61K 31/222, A61K 31/357, A61K 31/403, A61K 31/404, A61K 31/4045, A61K 31/4704, A61K 31/5377, A61P 17/02, A61P 23/00

(54) **BETA-BLOCKERS FOR TREATING AND/OR PREVENTING PATHOLOGICAL SCARS**
BETA-BLOCKER ZUR BEHANDLUNG UND/ODER PRÄVENTION VON PATHOLOGISCHEN NARBEN
BÊTA-BLOQUANTS POUR LE TRAITEMENT ET/OU LA PRÉVENTION DES CICATRICES PATHOLOGIQUES

(30) Priority: 27.12.2017 EP 17306940
(43) Date of publication of application: 04.11.2020
(73) Proprietor: Genepred Biotechnologies, 13006 Marseille (FR)
(72) Inventor: DESSEIN, Alain, 13009 MARSEILLE (FR)
(74) Representative: Lavoix
(86) International application number: PCT/EP2018/097028
(87) International publication number: WO 2019/129811

(56) References cited:
- EP-A1- 2 444 077
- WO-A1-2017/095236
- WO-A2-2009/015366
- ENOSHIRI TATSUKI ET AL: "[beta]-Adrenergic Receptor Blockers Reduce the Occurrence of Keloids and Hypertrophic Scars after Cardiac Device Implantation: A Single-Institution Case-Control Study.", PLASTIC AND RECONSTRUCTIVE SURGERY MAY 2017, vol. 139, no. 5, May 2017 (2017-05), pages 1248-1256, XP009505973, ISSN: 1529-4242
- DE MESQUITA ET AL: "About strawberry, crab claws, and the Sir James Black@?s invention. Hypothesis: Can we battle keloids with propranolol?", MEDICAL HYPOTHESES, EDEN PRESS, PENRITH, US, vol. 74, no. 2, 1 February 2010 (2010-02-01), pages 353-359, XP026816403, ISSN: 0306-9877 [retrieved on 2009-09-15]
- DATABASE EMBASE [Online] ELSEVIER SCIENCE PUBLISHERS, AMSTERDAM, NL; 1 March 2016 (2016-03-01), EL AYADI A ET AL: "Propranolol modulation of angiogenesis and its implication in hypertrophic scaring", XP002781899, Database accession no. EMB-619737378 & EL AYADI A ET AL: "Propranolol modulation of angiogenesis and its implication in hypertrophic scaring", WOUND REPAIR AND REGENERATION 20160301 BLACKWELL PUBLISHING INC. NLD, vol. 24, no. 2, 1 March 2016 (2016-03-01), pages A8 CONF 20160413 to 20160417 Atlanta, GA-28th Annu, ISSN: 1067-1927
- Ana M. Valdes ET AL: "Association of Beta-Blocker Use With Less Prevalent Joint Pain and Lower Opioid Requirement in People With Osteoarthritis : Beta-Blocker Use and OA Pain", Arthritis Care Research, vol. 69, no. 7, 7 June 2017 (2017-06-07), pages 1076-1081, XP055704454, US ISSN: 2151-464X, DOI: 10.1002/acr.23091

## Description

The present disclosure relates to the use of at least one beta-blocker for treating and/or preventing pathological scars, such as hypertrophic scars or keloids. Particularly, the present invention relates to the use of at least one beta-blocker which is propranolol, for reducing at least pair and/or itching related to keloids.

Pathological scars, such as hypertrophic scars and keloids, are characterized by an abnormality in wound healing, in which a fibrous tissue called "scar tissue" is formed during the process of skin wound healing, without regeneration of original normal tissue. While a hypertrophic scar occurs as a result of interference in wound healing, such as a large and deep wound, infection, contact with a foreign body or inappropriate suture, a keloid may arise from a very minor wound such as an insect bite or a puncture by a vaccine or just by skin irritation or itching. Keloids are characterized by their growth beyond the boundaries of the initial injury wound site. Hypertrophic scars usually regress within two years whereas keloids persist for years. However, hypertrophic scars and keloids are common in that both of their lesion portions are a red-colored elevated lesion, which is primarily characterized by an excessive accumulation of extracellular matrix and cell proliferation. The lesion portions are extremely hard, thereby markedly restricting the elasticity of the skin.

Keloids may cause a functional impediment if located over a joint, such as restriction of the range of the joint motion. Keloids are also accompanied by pain and itching, which could be moderate to severe, and which is very impairing for the afflicted patients. Scratching the scar causes small lesions which stimulate further the development of the scar. Scratching also result in increasing pain. Nevertheless pain may also occur at distance from the scar.

Therefore, the treatment of hypertrophic scars and keloids is important not only from cosmetic reasons, but also from the functional perspectives and to reduce pain and itching which are among the major complain of the patients. However, there is no appropriate model for animal experimentation for hypertrophic scars and keloids, thus the clarification of the etiology and pathology has not seen much progress up to present.

The treating methods of skin scars that are currently employed are the following:
- pressure therapy: in general, a sponge with an adhesive agent on one side is directly patched on the surface of the lesion portion, and this is pressed with surgical tape and fixed. In cases where the lesion portion is located on a movable part, on top of the surgical tape, the lesion portion is further wrapped with a dressing or supporter strap, or a girdle or corset is wore. Such pressuring flattens the lesion portion and relieves pain and itchiness. However, the termination of such pressuring results in re-swelling of the lesion portion and reemergence of pain and itchiness;
- surgical therapy: when the lesion portion is small or in the case of a narrow hypertrophic scar, the symptoms are relieved by excision thereof. However, the surgical therapy is not applicable to a wide lesion portion or hypertrophic scar extending over a large area, such as a thermal injury.
   Furthermore, in the case of a keloid, even if the lesion portion was once flattened, the lesion portion would reoccur therefrom, and would regenerate a lesion portion extending over an area larger than the one prior to the surgery. In order to prevent this recurrence, additional therapies after surgery are necessary, such as pressure therapy, radiation therapy or laser ablation. However, radiotherapy does nothing more than lowering the recurrence rate;
- silicone gel sheeting: hypertrophic scars and keloids have been treated by patching a silicone gel sheet, and in some cases, the symptoms are improved. The mechanism of action thereof is believed to be the moisturizing action; however, the details thereof are not clear. A hydrocolloid covering material has also been used in place of silicone gel. Yet, this therapy does nothing more than improving the symptoms and the effects thereof are not stable. In addition, there have been many cases where even an improvement of the symptom was not observed;
- pharmacotherapy: steroid drugs (such as triamcinolone acetonide) have been topically injected into the lesion portion of a hypertrophic scar and keloid. This injection not only flattens the lesion portion, but also fades the redness and alleviates the itchiness.

However, depending on the dosage, systemic side effects may occur. They thus cannot be administered over a prolonged period. Further, the injection is not applicable to lesions extending over a large area as well. In addition, since recurrence is observed in many patients upon the termination of steroid drug injection, steroid drugs are not an efficient treatment.

All these conventional therapies for hypertrophic scars and keloids, although called "treatment", are nothing more than supportive measures. That is, the conventional therapies do nothing more than simply flattening the lesion portion in a hypertrophic scar and keloid and temporarily improving the symptoms.

Thus, there still remains a need for treating a lesion tissue of hypertrophic scar or keloid efficiently. There is also a need for an efficient therapeutic agent, which
a) normalizes hypertrophic scars and keloids (i.e. which allows hypertrophic scars and keloids - at least in part - to recover to the normal tissue condition); and/or
b) reduce pain and itching associated with the scar; and/or
c) prevent the scar from developing i.e. after surgery.

The publication of Enoshiri et al (β-adrenergic receptor blockers reduce the occurrence of keloids and hypertrophic scars after cardiac device implantation: a single institution case-control study, Plastic and reconstructive surgery, p. 1248-1256, May 2017) suggests that β-adrenergic receptor blockers may be useful for reducing the occurrence of abnormal scars after cardiac device implantation. However, this document is not focused on keloids, and does not mention any effect on the symptoms of keloids either.

Surprisingly, the inventors have now discovered that the administration of propranolol to patients afflicted with keloids, is able to drastically reduce itching and pain in most patients. This reduction of pain and itching is observed in patients with keloids of all severity grades. Finally, the quality of life of the large majority of the tested patients is significantly improved. Propranolol may also help reducing the scar.

Propranolol is notably a beta-blocker well-known for more than fifty years.

Thus, the present invention relates to propranolol for use for reducing at least pain and/or itching related to keloids, wherein the keloids are of low or intermediate severity. The present disclosure relates to the use of propranolol for treating and/or preventing pathological scars (not part of the claimed invention).

Within the context of the disclosure, the term "treatment" denotes curative and/or symptomatic treatments. In particular, it can refer to improving the regression of the scar, reducing the progression/development of the scar, reducing or suppressing at least one of its symptoms or complications, or improving in any way the state of health of patients. Within the context of the disclosure, the term "prevention" denotes preventive treatments such as preventing the development of the scar and its pathological consequences.

Beta blockers, also known as beta-adrenergic blocking agents, constitute a very well-known therapeutic class. They are drugs that block norepinephrine and epinephrine (adrenaline) from binding to beta receptors on nerves. Norepinephrine and epinephrine are produced by nerves throughout the body as well as by the adrenal gland. They serve as neurotransmitters (chemicals that nerves use to communicate with one another) that may be active locally where they are produced, or elsewhere in the body, when they are released into the blood.

There are both alpha and beta receptors in the body. There are three types of beta receptors and they control several different functions based on their location in the body:
1. beta-1 (β1) receptors are located in the heart, eye, and kidneys;
2. beta-2 (β2) receptors are found in the lungs, gastrointestinal tract, liver, uterus, blood vessels and skeletal muscle; and
3. beta-3 (β3) receptors are located in fat cells.

By "beta-blocker", it is thus meant a compound which inhibits the binding of agonists, natural or artificial, to beta-adrenergic receptors of any type (beta-1, beta-2, beta-3 or others). According to the present disclosure, the beta-blocker may be a non-selective beta blocker, a beta-1-selective beta blocker, a beta-2-selective beta blocker or a mixture of alpha-1/beta-adrenergic antagonists. The beta-blocker may also be a mixture of two or more beta-blockers.

Examples of beta-blockers that may be used in the present disclosure are disclosed in Goodman and Gilman's the pharmacological basis of therapeutics, eleventh edition, chapter 10, pp 271-295, 2006.

Preferably, according to the present disclosure, the beta-blocker is a non-selective beta blocker.

Preferably, according to the present disclosure, the non-selective beta-blocker may be selected from propranolol, alprenolol, bucindolol, carteolol, carvedilol, labetalol, levobunolol, medroxalol, mepindolol, metipranolol, nadolol, oxprenolol, penbutolol, pindolol, sotalol, timolol, pharmaceutically acceptable salts thereof and mixtures thereof. When a beta-1 selective beta-blocker is used according to the present disclosure, it may be selected for example from the group comprising acebutolol, atenolol, betaxolol, bisoprolol, celiprolol, esmolol, metoprolol, nebivolol.

The beta-blocker according to the invention is propranolol or a pharmaceutically salt thereof, for example L- or D-propranolol or a mixture thereof. The mixture may be a mixture of L- and D-propranolol, with an amount thereof of 0:1 to 1:0, for example 1:1. Pharmaceutically acceptable salts of the propranolol may be propranolol chlorhydrate or any other preparation of propranolol, whether or not the preparation changes or alters the pharmacokinetic properties or metabolization of propranolol.

Thus, according to the disclosure (not part of the claimed invention), at least one beta-blocker is used for treating and/or preventing pathological scars.

Preferably, the pathological scars are chosen from keloids and hypertrophic scars. Contrary to hypertrophic scars, keloids are characterized by their growth beyond the boundaries of the initial injury wound site.

The hypertrophic scars may be caused by thermal or traumatic injury. The hypertrophic scars may be post-surgery scars, scars after burn injury, or scars caused by body piercings, cuts or pimples.

Preferably according to the disclosure, the beta-blocker is used for treating and/or preventing at least one of the symptoms of pathological scars. More preferably, the beta-blocker is used for treating and/or preventing at least one of the symptoms of keloids.

According to the invention, propranolol is used for reducing at least pain and/or itching related to keloids, wherein the keloids are of low or intermediate severity. Pain and/or itching may be the consequences of keloids, but also may be causative of keloids.

The keloid scars which are considered in the present invention are of low or intermediate severity.

Low severity generally corresponds to one or two scars on any part of the body. intermediate severity corresponds to various scars, including large cordons, on at least 3 different parts of the body.

Severe grades combine extended large patches and many long and large scars on different parts of the body.

More preferably according to the disclosure (not part of the claimed invention) the beta-blocker is used for treating keloids.

More preferably, the beta-blocker is used for improving the regression of keloids, and/or reducing or stopping the development of keloids.

More preferably according to the disclosure, the beta-blocker is used for preventing keloids. More preferably, the beta-blocker is used for preventing the development of keloids.

The present invention relates to propranolol for use for reducing at least pain and/or itching related to keloids, wherein the keloids are of low or intermediate severity.

The beta-blocker is usually included in a pharmaceutical composition (also called medicament). Said pharmaceutical composition comprises, in a pharmaceutically acceptable support, at least one beta-blocker according to the invention.

The amount of beta-blocker(s) in the composition according to the invention may vary in a broad range depending upon the patient, the mode of administration and the expected effect.

The compound or composition according to the invention can be administered orally or non-orally, for instance via topical, parenteral, intramuscular, intravenous, cutaneous, nasal or rectal route. Preferably it is administered orally or topically.

The pharmaceutical composition of the invention can present different forms including granules, powders, tablets, capsules, syrups, emulsions, suspensions, and forms used for non-oral administration, for instance injections, sprays, transdermal patches or suppositories. These pharmaceutical forms can be prepared via known conventional techniques.

For example, when the medicament is for oral administration, it may be in the form of a liquid formulation selected from the group comprising a solution, a syrup, a suspension, an emulsion and oral drops. When the medicament is in the form of an oral effervescent dosage form, it may be in a form selected from the group comprising tablets, granules and powders. When the medicament is the form of an oral powder or a multiparticulate system, it may be in a form selected from the group comprising beads, granules, mini-tablets and micro-granules. When the medicament is the form of an orodispersible dosage form, it may be in a form selected from the group comprising orodispersible tablets, lyophilised wafers, thin films, a chewable tablet, a tablet and a capsule and a medical chewing gum.

According to the present invention, the medicament may be for buccal and sublingual routes, for example selected from buccal and sublingual tablets, mucoadhesive preparations, lozenges, oromucosal drops and sprays. According to the present invention, the medicament may be for topical-transdermal administration, for example selected from ointments, creams, gels, lotions, patches and foams. According to the present invention, the medicament may be for nasal administration, for example selected from nasal drops, nasal sprays and nasal powders. According to the present invention, the medicament may be for rectal administration, for example a suppository or a hard gelatin capsule. According to the present invention, the medicament may be for parenteral administration, for example subcutaneous, intramuscular or intravenous administration.

The skilled person in the art understands clearly that the term "form" as used herein refers to the pharmaceutical formulation of the medicament for its practical use.

The preparation of an orally administered solid pharmaceutical form can be for instance performed by the following process: an excipient (for example lactose, sucrose, starch or mannitol), a desintegrant (for example calcium carbonate, calcium carboxymethylcellulose, alginic acid, sodium carboxymethylcellulose, colloidal silicon dioxide, sodium croscarmellose, crospovidone, guar gum, magnesium aluminium silicate, microcrystalline cellulose, cellulose powder, pregelatinised starch, sodium alginate or starch glycolate), a binder (for example alpha-starch, gum arabic, carboxymethylcellulose, polyvinylpyrrolidone, hydroxypropylcellulose, alginic acid, carbomer, dextrin, ethylcellulose, sodium alginate, maltodextrin, liquid glucose, magnesium aluminium silicate, hydroxyethylcellulose, methylcellulose or guar gum) and a lubricant (for example talc, magnesium stearate or polyethylene 6000) are added to the active principle and the mixture obtained is then tabletted. If necessary, the tablet can be coated via the known techniques, in order to mask the taste (for example with cocoa powder, mint, borneol or cinnamon powder) or to allow enteric dissolution or sustained release of the active principles. Coating products that can be used are, for example, ethylcellulose, hydroxymethylcellulose, polyoxyethylene glycol, cellulose acetophthalate, hydroxypropylmethylcellulose phthalate and Eudragit^{®} (methacrylic acid-acrylic acid copolymer), Opadry^{®} (hydroxypropylmethylcellulose + macrogol + titanium oxide + lactose monohydrate). Pharmaceutically acceptable colorants may be added (for example yellow iron oxide, red iron oxide or quinoline yellow lake).

Liquid pharmaceutical forms for oral administration include solutions, suspensions and emulsions. The aqueous solutions can be obtained by dissolving the active principle in water, followed by addition of flavorings, colorants, stabilizers and/or thickeners, if necessary. In order to improve the solubility, it is possible to add ethanol, propylene glycol or any other pharmaceutically acceptable non-aqueous solvent. The aqueous suspensions for oral use can be obtained by dispersing the finely divided active principle in water with a viscous product, such as a natural or synthetic gum or resin, methylcellulose or sodium carboxymethylcellulose.

The pharmaceutical forms for injection can be obtained, for example, by the following process: the active principle is dissolved, suspended or emulsified either in an aqueous medium (for example distilled water, physiological saline or Ringer's solution) or in an oily medium (for example olive oil, sesame seed oil, cottonseed oil, corn oil or propylene glycol), with a dispersant (for example Tween^{®} 80, HCO^{®} 60 (Nikko Chemicals), polyethylene glycol, carboxymethylcellulose or sodium alginate), a preserving agent (for example methyl p-hydroxybenzoate, propyl p-hydroxybenzoate, benzyl alcohol, chlorobutanol or phenol), an isotonicity agent (for example sodium chloride, glycerol, sorbitol or glucose) and optionally other additives, such as, if desired, a solubilizing agent (for example sodium salicylate or sodium acetate) or a stabilizer (for example human serum albumin).

Pharmaceutical forms for external use (topical use) can be obtained from a solid, semi-solid or liquid composition containing the active principle. For example, to obtain a solid form, the active principle can be treated with excipients (for example lactose, mannitol, starch, microcrystalline cellulose or sucrose) and a thickener (for example natural gums, cellulose derivatives or acrylic polymers) so as to convert them into powder. The liquid pharmaceutical compositions are prepared in substantially the same way as the forms for injection, as indicated previously. The semi-solid pharmaceutical forms are preferably in the form of aqueous or oily gels or in the form of pomades. These compositions may optionally contain a pH regulator (for example carbonic acid, phosphoric acid, citric acid, hydrochloric acid or sodium hydroxide) and a preserving agent (for example a p-hydroxybenzoic acid ester, chlorobutanol or benzalkonium chloride).

A method for the treatment and/or the prevention of a pathological scar in a subject, comprising administering to said subject at least one beta-blocker according to the invention, is also described herein, but is not part of the claimed invention.

In the present invention, the terms "subject" and "patient" are used indifferently and designate a mammal subject, preferably a human subject.

The amount of beta-blocker to be administered according to the invention may vary in a broad range depending upon the patient, the mode of administration and the expected effect. The regimen may be short or long. It may last for a few days until many years. In particular, the amount of beta-blocker may be comprised between 10 mg and 200 mg, with up to 3 daily intakes. Preferably, the amount of beta-blocker may be comprised between 20 mg and 100 mg, preferably between 30 and 60 mg, once daily.

The present invention is now illustrated by the following example and figures.

The figures are the following:
**Figure 1****. Effects of Propranolol on Itching in patients with different keloid grades**
**Figure 2****. Effect of Propranolol on Pain in patients with different Keloid grades**
**Figure 3****. Overall evaluation of the effect of the treatment on patient clinical conditions**

### Example: the effects of propranolol on Itching and pain caused by keloid scars

During a clinical evaluation and treatment of subjects infected with Schistosoma mansoni in Uganda, the inventors used propranolol to lower portal blood pressure in subjects with severe liver disease (hepatic fibrosis) and splenomegaly. High portal blood pressure causes the development of oesophageal varices and variceal bleedings that are the principal cause of death in S.mansoni-infected subjects. Propranolol is currently used to reduce the risk of variceal bleedings because that drug has blocking effects on beta receptors of the nervous system, thus reducing portal pressure. Five to ten percent of the 5000 fishermen and fisher women of the study had haemorrhagic bleedings from oesophageal varices or were at risk of bleeding. In an attempt to reduce bleedings, propranolol was given to them by the oral route, one 40 mg pill per day. This molecule has little side effects and can be prescribed safely for very long periods excepted to asthmatic subjects and to patients with cardiac problems.

During the study, one of the patients had mentioned that her keloid scars were not itching any more since she was treated for high portal pressure. This prompted the inventors to check whether this effect had also been noticed by other patients under the same treatment. The data presented here, have been obtained on 30 of these patients, they were collected from 30 out of 60 patients who had received this same treatment because of a high risk of variceal bleedings. The remaining 30 patients were not available, at that time, for evaluation of the propranolol effects.

### Material

The treatment of the patients with Propranolol is part of a large study the inventors are conducting on fishermen living on the banks of the Albert lake. This study, including Propranolol treatment, has been approved by the Uganda National Research Ethics committee (REC) and by the National Council for Science and Technology (agreement HS2256). The study is being carried out in accordance with Helsinky guidelines. Our ongoing work has been positively evaluated / controlled by onsite visit of the member of the Uganda Research Ethics Committee.

**Keloid Grading:** Patient keloid scars were classified as low, intermediate or high severity. Low severity corresponded to one or two scars on any part of the body, intermediate severity corresponded to various scars (including large cordons) on at least 3 different part of the body. Severe grades keloids combined extended large patches and many long and large scars on different part of the body.

**Statistical analysis** was performed using the SPSS software. The effects of the treatment were tested with the nonparametric Wilcoxon test performed on paired data (before and after treatment). P values were calculated for a two sided test.

### Results

### Propranolol reduces itching associated with keloids in most patients.

One third of the 30 patients reported moderate itching and two third severe itching annoying them very much for long periods (Table 1):

**Table 1. Pain and Itching associated with keloids in study subjects prior to treatment**

| | | | **Pain before Treatment** | | | **Total** |
|---|---|---|---|---|---|---|
| | | | **none** | **moderate** | **severe** | |
| **Itching before Treatment** | **moderate** | **n** | 2 | 5 | 3 | 10 |
| | | **%** | 20,0 | 50,0 | 30,0 | 100 |
| | **severe** | **n** | 4 | 9 | 7 | 20 |
| | | **%** | 20,0 | 45,0 | 35,0 | 100 |
| **Total Moderate + Severe** | | **n** | 6 | 14 | 10 | 30 |
| | | **%** | 20,0 | 467 | 33,3 | 100 |

The initial observation that oral administration of Propranol reduced keloid scar itching was confirmed by 80 % of the treated patients (Table 2):

**Table 2. Effect of propranolol on itching as a function of the itching intensity before treatment**

| | | | **Effect of Propranolol on Itching** | | | |
|---|---|---|---|---|---|---|
| | | | **none** | **partial** | **total** | |
| **Itching prior to Treatment** | **1** | **n** | **0** | **8** | **2** | **10** |
| | | % **in 1** | 0,0 | 80,0 | 20,0 | 100 |
| | | % **in none** / **partial** / **total** | 0 | 47,1 | 28,6 | 33,3 |
| | **2** | **n** | **6** | **9** | **5** | **20** |
| | | **% in 2** | 30,0 | 45,0 | 25,0 | 100 |
| | | % **in none** / **partial**/ **total** | 100 | 52,9 | 71,4 | 66,7 |
| **Global 1 + 2** | | **n** | **6** | **17** | **7** | **30** |
| | | **% in 1+2** | 20,0 | 56,7 | 23,3 | 100 |
| | | % **in none** / **partial**/ **total** | 100 | 100 | 100 | 100 |

They reported that taking 40 mg propranol per day for 2 to 4 days caused a marked (56.7%) or total (23.6%) reduction of itching of their keloid scars. Most patients reported that itching was so reduced that it was not annoying them anymore. Twenty percent of the patients reported no effect of the treatment on itching. The differences in Itching intensity before and after treatment was highly statistically significant (p<10-5, nonparametric, paired, two tailed Wilcoxon test). The inventors also used more stringent efficacy criteria considering that the drug had an effect only if itching had totally disappeared for at least 2 days. Using this more stringent criteria of efficacy, the inventors also found that itching differed significantly (p<10-2) before and after propranolol administration.

### Propranolol reduces the pain associated with keloids in most patients.

Patients with keloids have two major complains: severe itching and pain on the scars, in the scar periphery and eventually severe pain in any other parts of the body where no scars are located. Pain is often associated with itching or is secondary to scratching, it can also happen independently of itching; likewise, itching may not be associated with pain.

The distribution of pain and itching in the study patients is shown in Table 1. Eighty percent of the patients felt moderate to severe pain; 33.3% felt severe pain and severe itching; 46.7% felt severe pain and moderate itching.

Treatment with propranolol either reduced or totally abolished the pain associated with the keloids as shown in Table 3:

**Table 3. Effects of propranolol on Pain as a function of Pain intensity before treatment**

| | | | **Effects of Propranolol on Pain** | | | |
|---|---|---|---|---|---|---|
| | | | **none** | **partial** | **Total** | |
| **Pain Prior to Propranolol** | **1** | **n** | **4** | **5** | **5** | **14** |
| | | **% in 1** | 28,6 | 357 | 35,7 | 100 |
| | | **% in none** / **partial** / **total** | 66,7 | 45,5 | 71,4 | 58,3 |
| | **2** | **n** | **2** | **6** | **2** | **10** |
| | | **% in 2** | 20,0 | 60,0 | 20,0 | 100 |
| | | **% in none** / **partial** / **total** | 33,3 | 54,5 | 28,6 | 41,7 |
| **Global 1 + 2** | | **n** | **6** | **11** | **7** | **24** |
| | | **% in 1 + 2** | 25,0 | 45,8 | 29,2 | 100 |
| | | **% in none** / **partial** / **total** | 100 | 100 | 100 | 100 |

23.3 percent of study subjects reported a total abolition of pain for at least 2 to 3 days, 56.7% reported reduction of the pain and 20% reported no effects of the treatment on keloid associated pain. Comparison of pain before and after treatment (Wilcoxon test on paired data) indicated that the effect of the treatment was highly significant when considering full and partial effects (p<10-5) or when considering the full effect only (p<10-2) partial effect being taken as no effects.

**Reduction of itching and pain was observed in patients with keloids of all severity (low, intermediate or high) grades.**

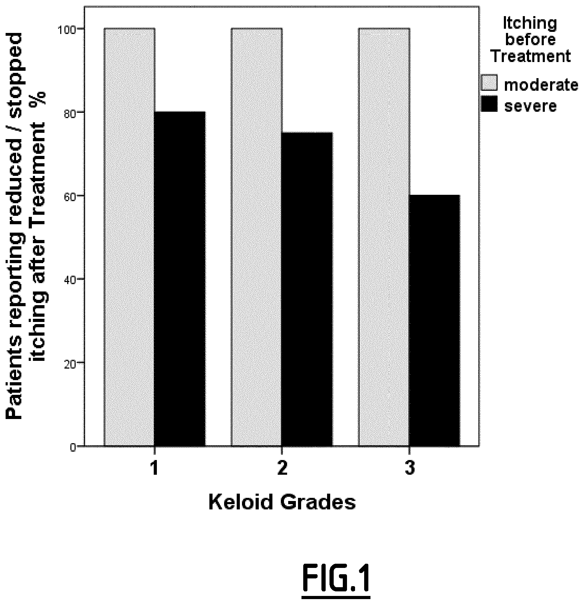

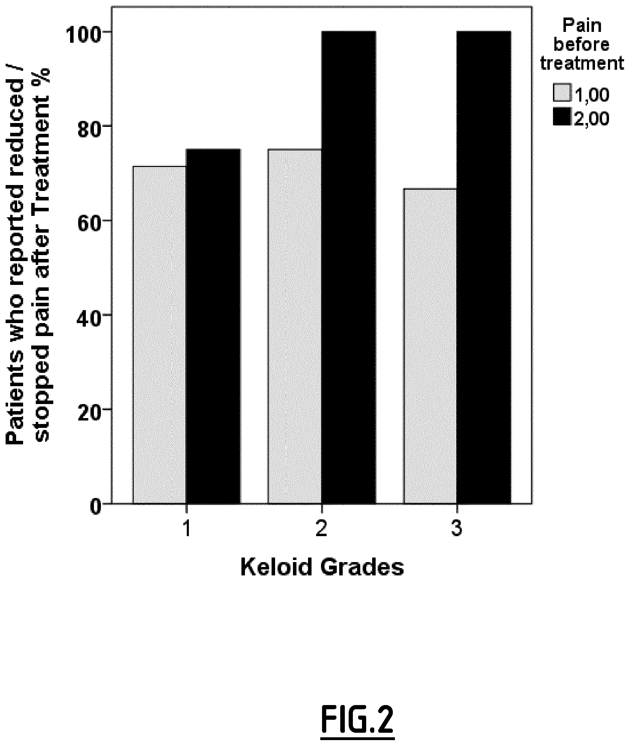

The effect of the treatment on itching and pain caused by keloids is shown in Figures 1 and 2 as a function of keloid grades. Propranolol treatment reduced pain and itching in patients with all keloid grades. Nevertheless, the data suggest that the treatment may have a more dramatic effect on low or intermediate grade keloids than on severe keloids.

The latter extend to large part of the body, covering entirely either the neck, the chess or the arms. This observation of a lessen effect on the very severe keloids was not statistically significant and must be evaluated on a larger study sample.

**Overall, 72.4 % of the patients estimated that propranolol had brought a very significant improvement of their clinical condition.**

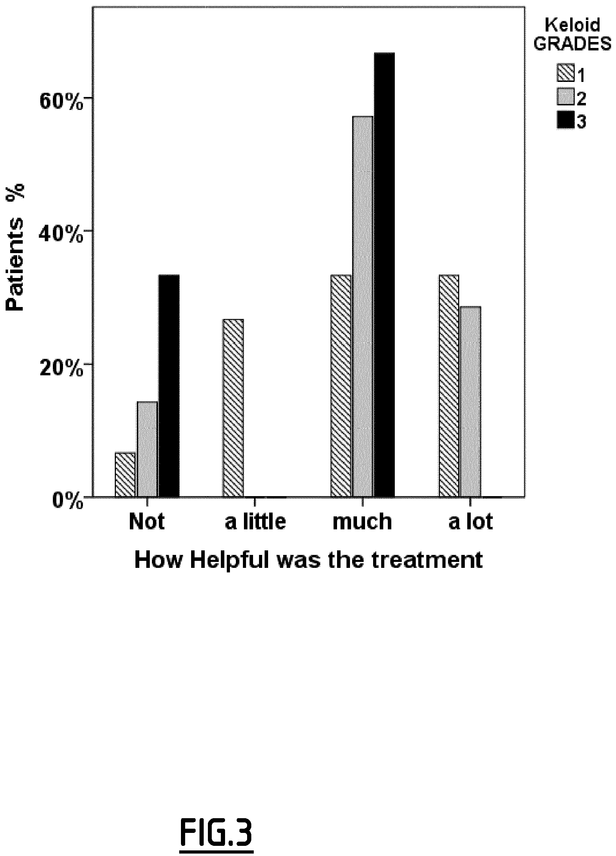

All patients were then asked to which extent, the treatment improved globally their living conditions by reducing the negative effects of keloids. The proposed answers were: a lot of improvement, much improvement, a little improvement and no improvement. Figure 3 shows the percentage of patients giving the possible answers. Only one answer per patient was accepted. Forty four (44.8%) and twenty seven (27.6%) percent of the patients estimated that the treatment had improved either much or a lot their condition, respectively. Then, a total of 72.4% of the patients felt that the treatment had clearly improved their clinical status.

## Claims

1. Propranolol for use for reducing at least pain and/or itching related to keloids, wherein the keloids are of low or intermediate severity.

2. Propranolol for use according to claim 1, wherein propranolol is administered orally or topically.

3. Propranolol for use according to any one of claims 1 to 2, wherein propranolol is administered in an amount comprised between 10 mg and 200 mg, with up to 3 daily intakes.

4. Propranolol for use according to any one of claims 1 to 3, wherein propranolol is administered in an amount comprised between 20 mg and 100 mg, once daily.

5. Propranolol for use according to any one of claims 1 to 2 or 4, wherein propranolol is administered in an amount comprised between 30 and 60 mg, once daily.

## Patentansprüche

1. Propranolol zur Verwendung zum Reduzieren von zumindest Schmerzen und/oder Juckreiz in Verbindung mit Keloiden, wobei die Keloide von geringem oder mittlerem Schweregrad sind.

2. Propranolol zur Verwendung nach Anspruch 1, wobei Propranolol oral oder topisch verabreicht wird.

3. Propranolol zur Verwendung nach einem der Ansprüche 1 bis 2, wobei Propranolol in einer Menge zwischen 10 mg und 200 mg mit bis zu 3 täglichen Einnahmen verabreicht wird.

4. Propranolol zur Verwendung nach einem der Ansprüche 1 bis 3, wobei Propranolol in einer Menge zwischen 20 mg und 100 mg einmal täglich verabreicht wird.

5. Propranolol zur Verwendung nach einem der Ansprüche 1 bis 2 oder 4, wobei Propranolol in einer Menge zwischen 30 und 60 mg einmal täglich verabreicht wird.

## Revendications

1. Propranolol destiné à une utilisation visant à réduire au moins la douleur et/ou les démangeaisons liées aux chéloïdes, les chéloïdes étant de gravité faible ou moyenne.

2. Propranolol destiné à une utilisation selon la revendication 1, dans lequel le propranolol est administré par voie orale ou topique.

3. Propranolol destiné à une utilisation selon l'une quelconque des revendications 1 et 2, dans lequel le propranolol est administré dans une quantité comprise entre 10 mg et 200 mg, avec jusqu'à 3 prises quotidiennes.

4. Propranolol destiné à une utilisation selon l'une quelconque des revendications 1 à 3, dans lequel le propranolol est administré dans une quantité comprise entre 20 mg et 100 mg, une fois par jour.

5. Propranolol destiné à une utilisation selon l'une quelconque des revendications 1 et 2 ou 4, dans lequel le propranolol est administré dans une quantité comprise entre 30 et 60 mg, une fois par jour.
